# EUROPEAN PATENT APPLICATION

(11) **EP 4 424 667 A1**
(43) Date of publication of application: **04.09.2024**
(21) Application number: 22886760.2
(22) Date of filing: 17.10.2022
(51) Int. Cl.: C07D 201/12, C07D 223/10, C08G 69/16

(54) **METHOD FOR PRODUCING E-CAPROLACTAM AND METHOD FOR PRODUCING POLYAMIDE 6**

(30) Priority: 29.10.2021 JP 2021178279
(71) Applicant: Toray Industries, Inc., Tokyo 103-8666 (JP)
(72) Inventor: YAMASHITA, Kohei, Nagoya-shi, Aichi 455-8502 (JP); TAKAHASHI, Akihiro, Nagoya-shi, Aichi 455-8502 (JP); KATO, Masashi, Tokai-shi, Aichi 476-8567 (JP); NISHIMURA, Mihoko, Nagoya-shi, Aichi 455-8502 (JP); KATO, Koya, Nagoya-shi, Aichi 455-8502 (JP)
(74) Representative: Mewburn Ellis LLP
(86) International application number: PCT/JP2022/038576
(87) International publication number: WO 2023/074432

(57) **Abstract**

To provide a method for producing ε-caprolactam from a polyamide 6 resin composition, the method achieving both circulation utilization of fossil resources and reduction in global warming gas emissions, and therefore being green and energy-saving. The present invention is a method for producing ε-caprolactam, including bringing a resin composition (A) containing at least polyamide 6 and a polyamide 6 oligomer aqueous solution (C) heated to 290°C or higher and 350°C or lower into contact with each other, or bringing a resin composition (A) containing at least polyamide 6, a polyamide 6 oligomer aqueous solution (C) heated to 290°C or higher and 350°C or lower, and further water (B) heated to 290°C or higher and 350°C or lower into contact with each other.

## Description

### Field

The present invention relates to a method for decomposing polyamide 6, which achieves both circulation utilization of fossil resources and reduction in global warming gas emissions, and more particularly to a recycling method for collecting ε-caprolactam with high purity in high yield by depolymerizing polyamide 6 using a small amount of water having a high specific heat capacity and a high vaporization heat and an aqueous solution containing a linear polyamide 6 oligomer.

### Background

In recent years, with the ocean plastic problem as a trigger, an interest in global environmental issues has increased, and there is a growing recognition that construction of a sustainable society is required. Global environmental issues include global warming, resource depletion, water shortage, and the like, and most of them are caused by an increase in resource consumption and global warming gas emissions due to rapid human activities after the industrial revolution. Therefore, in order to construct a sustainable society, technologies related to circulation utilization of fossil resources such as plastics and reduction in global warming gas emissions are increasingly important.

As a plastic recycling technology, a technology for conversion into a pyrolysis oil or gas by thermally decomposing a plastic waste material and collecting a gas, an oil, or the like has attracted attention, and many methods have been proposed. For example, Patent Literature 1 discloses a method for producing a hydrocarbon by a process including thermal decomposition and steam cracking of a waste plastic. These methods have an advantage that mixed waste plastics can be converted into a pyrolysis oil but cracking at a high temperature of 800°C or higher is required in order to convert a pyrolysis oil into a secondary raw material such as a plastic monomer, and further, when, for example, a plastic containing chlorine such as polyvinyl chloride or sulfur such as polyarylene sulfide is mixed in waste plastics, there is a problem of plant corrosion, and when a plastic containing oxygen and nitrogen such as a polyamide is mixed therein, there is a concern about explosion.

As a method for recycling polyamide 6, which is used in a large amount in various fields as a fiber, a film, and an engineering plastic, a method for obtaining ε-caprolactam, which is a raw material, by blowing superheated steam in the presence of a phosphoric acid catalyst is disclosed (see, for example, Patent Literature 2) .

In addition, as a method for depolymerizing polyamide 6 without using a catalyst such as an acid or a base, a method for collecting a lactam by bringing polyamide 6 and heated water into contact with each other at a temperature of 280°C to 320°C is disclosed (see, for example, Patent Literatures 3 and 4.).

### Citation List

### Patent Literature

Patent Literature 1: JP 2019-533041 A
Patent Literature 2: JP H08-217746 A
Patent Literature 3: JP H10-510280 A
Patent Literature 4: JP H10-510282 A

### Summary

### Technical Problem

The method for collecting ε-caprolactam disclosed in Patent Literature 2 is a high-yield reaction in which the depolymerization yield of polyamide 6 is 80% or more, but requires a long time for the depolymerization reaction. Further, since a large amount of superheated water vapor, which is about 10 times the amount of nylon 6 fibers, is required, the technology still has a problem in achieving both circulation utilization of fossil resources and global warming gas emissions. In addition, since this method is a reaction using phosphoric acid as a catalyst, the reaction is susceptible to impurities such as catalyst deactivation due to an additive contained in a plastic or adhering impurities in a waste plastic. In fact, the present inventors conducted a depolymerization experiment using polyamide 6 containing a potassium salt as a raw material under the same or similar conditions to the method described in Patent Literature 2, and as a result, it was found that the yield of ε-caprolactam significantly decreases. This is considered to be due to deactivation of the phosphoric acid catalytic action by the potassium salt.

Meanwhile, in the methods for collecting ε-caprolactam disclosed in Patent Literatures 3 and 4, only water is used in the depolymerization reaction, and a catalyst such as phosphoric acid is not used, so that there is an advantage that reaction deactivation due to an additive, adhering impurities, or the like does not occur. However, in the disclosed method for collecting ε-caprolactam, water having a specific heat capacity of 4.2 kJ/kg·K and a vaporization heat of 2,250 kJ/kg, which are very high, is used in an amount about 10 times the amount of polyamide 6, which is large, to carry out a reaction for a long time, and thus a large amount of energy is required in the depolymerization reaction and the collection of ε-caprolactam from a low concentration ε-caprolactam aqueous solution. In addition, even when the amount of water used was simply reduced under the same conditions or similar conditions, the collection rate of ε-caprolactam only decreased. This is considered to be because the thermodynamic equilibrium point of ε-caprolactam generated by depolymerization and a linear oligomer generated by hydrolytic ring-opening of ε-caprolactam moved to the linear oligomer side only by simply reducing the amount of water used.

### Solution to Problem

In order to solve the above problems, the present invention has the following configurations.
1. A method for producing ε-caprolactam, including bringing a resin composition (A) containing at least polyamide 6 and a polyamide 6 oligomer aqueous solution (C) heated to 290°C or higher and 350°C or lower into contact with each other, or bringing a resin composition (A) containing at least polyamide 6, a polyamide 6 oligomer aqueous solution (C) heated to 290°C or higher and 350°C or lower, and further water (B) heated to 290°C or higher and 350°C or lower into contact with each other.
2. The method for producing ε-caprolactam according to item 1, wherein the polyamide 6 oligomer aqueous solution (C) is further added in a method for producing ε-caprolactam by adding the resin composition (A) containing at least polyamide 6 and the water (B) heated to 290°C or higher and 350°C or lower to make contact with each other.
3. The method for producing ε-caprolactam according to item 1 or 2, wherein a polyamide 6 oligomer used in the polyamide 6 oligomer aqueous solution (C) is a polyamide 6 oligomer by-produced in the method for producing ε-caprolactam.
4. The method for producing ε-caprolactam according to any one of items 1 to 3, wherein an amount of a linear 2-to 12-mer oligomer contained in the polyamide 6 oligomer is 90 mass% or more.
5. The method for producing ε-caprolactam according to any one of items 1 to 4, wherein the contact is made under a condition that a product of X and Y is 2,000 or less when a mass ratio of water to a sum of polyamide 6 and a polyamide 6 oligomer used in the method for producing ε-caprolactam is represented by X : 1 and a reaction temperature is represented by Y°C.
6. The method for producing ε-caprolactam according to any one of items 1 to 5, wherein the resin composition (A) containing at least polyamide 6 contains an alkali metal halide.
7. The method for producing ε-caprolactam according to any one of items 1 to 6, wherein the polyamide 6 oligomer aqueous solution (C) is an extract liquid obtained in a step of extracting a polyamide 6 oligomer with hot water from polyamide 6 which is a product at a time of production of polyamide 6.
8. The method for producing ε-caprolactam according to any one of items 1 to 7, wherein the resin composition (A) containing at least polyamide 6 is a waste of a resin molded body containing at least polyamide 6.
9. A method for producing polyamide 6, including obtaining ε-caprolactam by the method according to any one of items 1 to 8, and polymerizing polyamide 6. Advantageous Effects of Invention

The present invention can provide a method for producing ε-caprolactam with a small energy consumption because ε-caprolactam can be produced in high yield and high selectivity even when a small amount of water having a high specific heat capacity is used in the depolymerization of a polyamide 6 resin composition.

### Description of Embodiments

Hereinafter, the present invention will be described in more detail.

### (1) Resin composition (A)

The present invention is a method for producing ε-caprolactam characterized in that a polyamide 6 oligomer aqueous solution (C) is further added when water (B) heated to 290°C or higher and 350°C or lower is added and brought into contact with a resin composition (A) containing at least polyamide 6.

The polyamide 6 used in the present invention is a polyamide resin in which 6-aminocaproic acid and/or ε-caprolactam is used as a main raw material. The polyamide 6 may be one obtained by copolymerization with another monomer as long as the object of the present invention is not impaired. Here, the phrase "used as a main raw material" means that a total of 50 mol% or more of a unit derived from 6-aminocaproic acid or a unit derived from ε-caprolactam is contained in a total of 100 mol% of monomer units included in the polyamide resin. A unit derived from 6-aminocaproic acid or a unit derived from ε-caprolactam is more preferably contained in an amount of 70 mol% or more, and still more preferably 90 mol% or more.

Examples of another monomer to be copolymerized include amino acids such as 11-aminoundecanoic acid, 12-aminododecanoic acid, and para-aminomethylbenzoic acid, lactams such as ω-laurolactam, aliphatic diamines such as tetramethylenediamine, pentamethylenediamine, hexamethylenediamine, 2-methylpentamethylenediamine, nonamethylenediamine, decamethylenediamine, undecamethylenediamine, dodecamethylenediamine, 2,2,4-/2,4,4-trimethylhexamethylenediamine, and 5-methylnonamethylenediamine, aromatic diamines such as metaxylylenediamine and para-xylylenediamine, alicyclic diamines such as 1,3-bis(aminomethyl)cyclohexane, 1,4-bis(aminomethyl)cyclohexane, 1-amino-3-aminomethyl-3,5,5-trimethylcyclohexane, bis(4-aminocyclohexyl)methane, bis(3-methyl-4-aminocyclohexyl)methane, 2,2-bis(4-aminocyclohexyl)propane, 1,4-bis(3-aminopropyl)piperazine, and 1-(2-aminoethyl)piperazine, aliphatic dicarboxylic acids such as adipic acid, suberic acid, azelaic acid, sebacic acid, and dodecanedioic acid, aromatic dicarboxylic acids such as terephthalic acid, isophthalic acid, 2-chloroterephthalic acid, 2-methylterephthalic acid, 5-methylisophthalic acid, 5-sodium sulfoisophthalate, and 2,6-naphthalenedicarboxylic acid, and alicyclic dicarboxylic acids such as 1,4-cyclohexanedicarboxylic acid, 1,3-cyclohexanedicarboxylic acid, 1,2-cyclohexanedicarboxylic acid, and 1,3-cyclopentanedicarboxylic acid. Two or more of these may be copolymerized.

In addition, a polymerization degree regulator, a terminal group regulator or the like may be added to such polyamide 6. Examples of the polymerization degree regulator and the terminal group regulator include acetic acid and benzoic acid.

The polymerization degree of the polyamide 6 of the present invention is not particularly limited, but the relative viscosity measured at 25°C in a 98% concentrated sulfuric acid solution having a resin concentration of 0.01 g/mL is preferably in a range of 1.5 to 5.0. The relative viscosity in such a preferable range can be preferably exemplified because the reaction efficiency with a small amount of water tends to increase.

The polyamide 6 of the present invention may contain a cyclic oligomer represented by the following formula (a). The amount of the cyclic oligomer represented by the following formula (a) contained in the polyamide 6 is not particularly limited, but can be exemplified by preferably 2.0 mass% or less, more preferably 1.8 mass% or less, and still more preferably 1.5 mass% or less. In the cyclic oligomer represented by the following formula (a), m is an integer of 2 to 4. Since the cyclic oligomer represented by the following formula (a) melts and volatilizes to cause line blockage or the like, when the amount of the cyclic oligomer is in the preferable range, there is a tendency that line blockage due to melting and volatilization can be prevented. Note that the cyclic oligomer represented by the following formula (a) in which m is 5 or more is not the focus of the present invention in consideration of the degree of volatilization thereof.

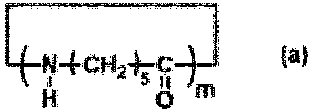

The resin composition (A) of the present invention may further contain an alkali metal halide as long as the object of the present invention is not impaired. Examples of the alkali metal halide include alkali metal halides such as lithium iodide, sodium iodide, potassium iodide, lithium bromide, sodium bromide, potassium bromide, lithium chloride, sodium chloride, and potassium chloride, and two or more of these can be used in combination. Among them, potassium iodide is preferable from the viewpoint of easy availability, excellent dispersibility in polyamide 6, higher reactivity with radicals, and further improvement of retention stability at a high temperature. Further, these alkali metal halides are more preferably used in combination with a Group 11 metal halide such as copper(I) iodide, copper(I) bromide, or copper(I) chloride because the retention stability at a high temperature is further improved.

Such an alkali metal halide is preferably blended in an amount of 0.01 to 1 part by mass with respect to 100 parts by mass of polyamide 6. By blending the alkali metal halide in such a preferable range, side reactions other than hydrolysis in the present process can be prevented, and the lactam yield tends to increase. The blending amount of the alkali metal halide is more preferably 0.02 to 0.5 parts by mass, and still more preferably 0.03 to 0.4 parts by mass.

The resin composition (A) of the present invention may contain a fibrous filling material. The fibrous filling material here may be any filling material having a fibrous shape. Specific examples thereof include glass fibers, polyacrylonitrile (PAN)-based or pitch-based carbon fibers, metal fibers such as stainless steel fibers, aluminum fibers, and brass fibers, organic fibers such as polyester fibers and aromatic polyamide fibers, gypsum fibers, ceramic fibers, asbestos fibers, zirconia fibers, alumina fibers, silica fibers, titanium oxide fibers, silicon carbide fibers, rock wool, potassium titanate whiskers, silicon nitride whiskers, fibrous or whisker-like filling materials of wollastonite, alumina silicate, and the like, and glass fibers, carbon fibers, aromatic polyamide fibers, and polyester fibers coated with one or more metals selected from the group consisting of nickel, copper, cobalt, silver, aluminum, iron, and alloys thereof. Two or more of these may be contained. The content of the fibrous filling material is preferably 1 to 200 parts by mass with respect to 100 parts by mass of the resin composition (A).

In the resin composition (A) of the present invention, a filler other than the fibrous filling material, a thermoplastic resin other than polyamide 6, various additives, or the like can be further blended as long as the object of the present invention is not impaired. The filler other than the fibrous filling material may be either an organic filler or an inorganic filler, and examples thereof include non-fibrous filling materials, and two or more of these may be blended. Examples of the non-fibrous filling material include non-swellable silicates such as talc, wollastonite, zeolite, sericite, mica, kaolin, clay, pyrophyllite, bentonite, asbestos, alumina silicate, and calcium silicate, swellable layered silicates such as Li-type fluoroteniolite, Na-type fluoroteniolite, Na-type tetrasilicon fluorine mica, and Li-type tetrasilicon fluorine mica, metal oxides such as silicon oxide, magnesium oxide, alumina, silica, diatomaceous earth, zirconium oxide, titanium oxide, iron oxide, zinc oxide, calcium oxide, tin oxide, and antimony oxide, metal carbonates such as calcium carbonate, magnesium carbonate, zinc carbonate, barium carbonate, dolomite, and hydrotalcite, metal sulfates such as calcium sulfate and barium sulfate, metal hydroxides such as magnesium hydroxide, calcium hydroxide, aluminum hydroxide, and basic magnesium carbonate, smectite-based clay minerals such as montmorillonite, beidellite, nontronite, saponite, hectorite, and sauconite, various clay minerals such as vermiculite, halloysite, kanemite, kenyaite, zirconium phosphate, and titanium phosphate, glass beads, glass flakes, ceramic beads, boron nitride, aluminum nitride, silicon carbide, calcium phosphate, carbon black, and graphite. In the swellable layered silicate described above, an exchangeable cation present between layers may be exchanged with an organic onium ion. Examples of the organic onium ion include an ammonium ion, a phosphonium ion, and a sulfonium ion.

Specific examples of the various additives include a heat stabilizer such as a phenolic compound such as N,N'-hexamethylenebis(3,5-di-t-butyl-4-hydroxyhydrocinnamide) or tetrakis[methylene-3-(3',5'-di-t-butyl-4'-hydroxyphenyl)propionate]methane, a phosphorus-based compound, a sulfur-based compound such as a mercaptobenzimidazole-based compound, a dithiocarbamic acid-based compound, or an organic thioacid-based compound, or an amine-based compound such as N,N'-di-2-naphthyl-p-phenylenediamine or 4,4'-bis(α,α-dimethylbenzyl)diphenylamine, a coupling agent such as an isocyanate-based compound, an organic silane-based compound, an organic titanate-based compound, an organic borane-based compound, or an epoxy compound, a plasticizer such as a polyalkylene oxide oligomer-based compound, a thioether-based compound, an ester-based compound, or an organic phosphorus-based compound, a crystal nucleating agent such as an organic phosphorus compound or polyether ether ketone, a metal soap such as a montanic acid wax, lithium stearate, or aluminum stearate, a release agent such as ethylenediamine-stearic acid-sebacic acid polycondensate or a silicone-based compound, an anti-coloring agent such as a hypophosphite, a lubricant, an ultraviolet light inhibitor, a colorant, a flame retardant, and a foaming agent. When such an additive is contained, the content thereof is preferably 10 parts by mass or less, and more preferably 1 part by mass or less with respect to 100 parts by mass of polyamide 6.

Specific examples of the thermoplastic resin other than polyamide 6 contained in the resin composition (A) include a polyamide resin other than polyamide 6, a polyester resin, a polyolefin resin, a modified polyphenylene ether resin, a polysulfone resin, a polyketone resin, a polyetherimide resin, a polyarylate resin, a polyethersulfone resin, a polyetherketone resin, a polythioetherketone resin, a polyetheretherketone resin, a polyimide resin, a polyamideimide resin, a tetrafluorinated polyethylene resin, and a polyphenylene sulfide resin. Two or more of these may be blended. Further, it can be preferably exemplified that the blending amount of the thermoplastic resin other than polyamide 6 here is set to 30 parts by mass or less with respect to 100 parts by mass of polyamide 6 in the thermoplastic resin (A) of the present invention.

The resin composition (A) containing polyamide 6 of the present invention may be a waste of a resin molded body containing at least polyamide 6. Examples of the waste of the resin molded body containing polyamide 6 include a polyamide 6 product, an industrial waste generated in the process for producing a polyamide 6 product, and a used polyamide 6 product waste. Examples of the polyamide 6 product include fiber structures for clothing such as old clothes, uniforms, sportswear, and inner wear, industrial fiber structures such as curtains, carpets, ropes, nets, belts, and sheets, molded parts for residential building materials, electrical and electronic molded parts, aircraft parts, industrial machine parts, film products, extrusion molded products, in situ polymerized molded products, and RIM molded products. In addition, product scraps, pellet scraps, block scraps, cutting scraps during cutting work, and the like generated in these production steps also serve as waste targets.

### (2) Polyamide 6 oligomer

The method for producing ε-caprolactam of the present invention is characterized by bringing a polyamide 6 oligomer aqueous solution (C) heated to 290°C or higher and 350°C or lower into contact with a resin composition (A) containing at least polyamide 6, or by bringing a resin composition (A) containing at least polyamide 6, a polyamide 6 oligomer aqueous solution (C) heated to 290°C or higher and 350°C or lower, and further water (B) heated to 290°C or higher and 350°C or lower into contact with each other.

The polyamide 6 oligomer here is a polyamide 6 oligomer in which 6-aminocaproic acid and/or ε-caprolactam is contained as a main component. Another monomer may be contained as long as the object of the present invention is not impaired. The phrase "contained as a main component" here means that a total of 50 mol% or more of a unit derived from 6-aminocaproic acid or a unit derived from ε-caprolactam is contained in a total of 100 mol% of monomer units included in the polyamide 6 oligomer. A unit derived from 6-aminocaproic acid or a unit derived from ε-caprolactam is more preferably contained in an amount of 70 mol% or more, and still more preferably 90 mol% or more.

Examples of another monomer contained in the polyamide 6 oligomer include amino acids such as 11-aminoundecanoic acid, 12-aminododecanoic acid, and para-aminomethylbenzoic acid, lactams such as ω-laurolactam, aliphatic diamines such as tetramethylenediamine, pentamethylenediamine, hexamethylenediamine, 2-methylpentamethylenediamine, nonamethylenediamine, decamethylenediamine, undecamethylenediamine, dodecamethylenediamine, 2,2,4-/2,4,4-trimethylhexamethylenediamine, and 5-methylnonamethylenediamine, aromatic diamines such as meta-xylylenediamine and para-xylylenediamine, alicyclic diamines such as 1,3-bis(aminomethyl)cyclohexane, 1,4-bis(aminomethyl)cyclohexane, 1-amino-3-aminomethyl-3,5,5-trimethylcyclohexane, bis(4-aminocyclohexyl)methane, bis(3-methyl-4-aminocyclohexyl)methane, 2,2-bis(4-aminocyclohexyl)propane, 1,4-bis(3-aminopropyl)piperazine, and 1-(2-aminoethyl)piperazine, aliphatic dicarboxylic acids such as adipic acid, suberic acid, azelaic acid, sebacic acid, and dodecanedioic acid, aromatic dicarboxylic acids such as terephthalic acid, isophthalic acid, 2-chloroterephthalic acid, 2-methylterephthalic acid, 5-methylisophthalic acid, 5-sodium sulfoisophthalate, and 2,6-naphthalenedicarboxylic acid, and alicyclic dicarboxylic acids such as 1,4-cyclohexanedicarboxylic acid, 1,3-cyclohexanedicarboxylic acid, 1,2-cyclohexanedicarboxylic acid, and 1,3-cyclopentanedicarboxylic acid. Two or more of these may be contained.

The number average molecular weight of the polyamide 6 oligomer of the present invention is not particularly limited, but it can be exemplified that the number average molecular weight is preferably in a range of 100 to 5,000, more preferably in a range of 200 to 3,000, and particularly preferably in a range of 200 to 2,000. When the molecular weight of the polyamide 6 oligomer is in such a preferable range, the solubility in water is increased, and a polyamide 6 oligomer aqueous solution (B) used in the present invention tends to be easily prepared. The number average molecular weight here was calculated by GPC analysis using 1,1,1,3,3,3-hexafluoro-2-propanol as a solvent. GPC-HFIP-805 manufactured by Showa Denko K.K. was used as a column, and PMMA was used as a standard substance.

Further, the composition of the polyamide 6 oligomer used in the present invention is not particularly limited, but it can be preferably exemplified that the content of a 2- to 12-mer linear polyamide 6 oligomer contained in the polyamide 6 oligomer is 90% mass% or more. It can be exemplified that the content of a linear 2- to 12-mer oligomer is more preferably 93 mass% or more, and particularly preferably 95 mass% or more. When the content of a 2- to 12-mer linear polyamide 6 oligomer contained in the polyamide 6 oligomer is in such a preferable range, the solubility in water is increased, and further the concentration of the terminal carboxylic acid in the polyamide 6 oligomer is increased, so that the reaction of polyamide 6 with water is promoted, and the production efficiency of ε-caprolactam tends to increase. The amount of a linear 2- to 12-mer oligomer in the polyamide 6 oligomer here was quantitatively analyzed by high-performance liquid chromatography using a formic acid aqueous solution and a formic acid acetonitrile solution as eluents.

The method for preparing the polyamide 6 oligomer used in the present invention is not particularly limited, and for example, a polyamide 6 oligomer contained in an extract liquid when a polyamide 6 resin is subjected to hot water extraction at the time of production of a general fatty acid-based polyamide 6 resin, or a polyamide 6 oligomer prepared by the same method as a method for synthesizing a general fatty acid-based polyamide 6 resin may be used. In addition, a polyamide 6 oligomer obtained as a by-product when ε-caprolactam is produced by bringing a resin composition containing at least polyamide 6 and water heated to 290°C or higher and 350°C or lower into contact with each other may be used. Still further, a polyamide 6 oligomer obtained as a by-product when ε-caprolactam is produced by bringing a resin composition (A) containing at least polyamide 6 and a polyamide 6 oligomer aqueous solution (C) heated to 290°C or higher and 350°C or lower into contact with each other, or by bringing a resin composition (A) containing at least polyamide 6, a polyamide 6 oligomer aqueous solution (C) heated to 290°C or higher and 350°C or lower, and further water (B) heated to 290°C or higher and 350°C or lower into contact with each other. From the viewpoint of reducing an industrial waste in production of ε-caprolactam, it is preferable to use a polyamide 6 oligomer collected as a by-product when ε-caprolactam is produced by bringing water heated to 290°C or higher and 350°C or lower into contact, a by-product when ε-caprolactam is produced by bringing a resin composition (A) containing at least polyamide 6 and a polyamide 6 oligomer aqueous solution (C) heated to 290°C or higher and 350°C or lower into contact with each other, or, a by-product when ε-caprolactam is produced by bringing a resin composition (A) containing at least polyamide 6, a polyamide 6 oligomer aqueous solution (C) heated to 290°C or higher and 350°C or lower, and further water (B) heated to 290°C or higher and 350°C or lower into contact.

### (3) Polyamide 6 oligomer aqueous solution (C)

The polyamide 6 oligomer aqueous solution (C) used in the present invention is prepared by heating and mixing the polyamide 6 oligomer and water.

The water used when the polyamide 6 oligomer aqueous solution (C) is prepared is not particularly limited, and tap water, ion exchanged water, distilled water, well water, or the like can be used, but ion exchanged water or distilled water is preferably used from the viewpoint of preventing side reactions due to the influence of coexisting salts.

In addition, the concentration of the polyamide 6 oligomer in the polyamide 6 oligomer aqueous solution (C) used in the present invention may be any concentration as long as the polyamide 6 oligomer is dissolved in water when heated to 290°C or higher and 350°C or lower, but can be preferably exemplified by 20 mass% or less, more preferably 15 mass% or less, and still more preferably 10 mass% or less. When the concentration of the polyamide 6 oligomer is in such a preferable range, solubility in water when the polyamide 6 oligomer aqueous solution (C) is prepared is increased, and the polyamide 6 oligomer aqueous solution (C) can be prepared at a lower temperature.

Further, the extract liquid containing a polyamide 6 oligomer in the step of extracting a polyamide 6 oligomer with hot water from polyamide 6, which is a product at the time of production of polyamide 6, can also be used as the polyamide 6 oligomer aqueous solution (C). Usually, a polyamide 6 resin obtained by polymerizing ε-caprolactam contains unreacted monomers generated in a polymerization equilibrium reaction and polyamide 6 oligomers as impurities, and in order to remove them, a pellet after polymerization is supplied to a hot water extraction tower, and unreacted monomers and polyamide 6 oligomers are extracted and removed by hot water extraction. Use of the extract liquid in the step of extracting a polyamide 6 oligomer with hot water from polyamide 6 at the time of production of polyamide 6 as the polyamide 6 oligomer aqueous solution (C) of the present invention can also be exemplified as a preferable form from the viewpoint of industrial waste reduction.

### (4) Method for producing ε-caprolactam

The method for producing ε-caprolactam of the present invention is characterized by bringing a resin composition (A) containing at least polyamide 6 and a polyamide 6 oligomer aqueous solution (C) heated to 290°C or higher and 350°C or lower into contact with each other, or by adding water (B) heated to 290°C or higher and 350°C or lower to a resin composition (A) containing at least polyamide 6 and a polyamide 6 oligomer aqueous solution (C) heated to 290°C or higher and 350°C or lower.

In the method for producing ε-caprolactam of the present invention, polyamide 6 is hydrolyzed with water in the polyamide 6 oligomer aqueous solution (C) heated to 290°C or higher and 350°C or lower, or water in the polyamide 6 oligomer aqueous solution (C) heated to 290°C or higher and 350°C or lower, and water heated to 290°C or higher and 350°C or lower. When the pressure is increased to 22.1 MPa and the temperature is increased to 374.2°C, water shows a state of being not a liquid or a gas. This point is referred to as a critical point of water, and hot water having a temperature and a pressure lower than the critical point is referred to as subcritical water. Although the subcritical water is water, the subcritical water has characteristics that (i) the permittivity is low and (ii) the ion product is high, and the permittivity and the ion product of the subcritical water depend on the temperature and the partial pressure of water and can be controlled. Due to its low permittivity, the subcritical water serves as an excellent solvent for an organic compound even though it is water, and due to its high ion product, the hydrogen ion and hydroxide ion concentrations are increased, so that the subcritical water has an excellent hydrolysis action. The temperature of the water (B) and the polyamide 6 oligomer aqueous solution (C) of the present invention is preferably 300°C or higher and 340°C or lower, and more preferably 320°C or higher and 340°C or lower. When the temperature is in such a preferable range, there is a tendency that device corrosion during reaction can be prevented. Further, the pressure of water in the case of using the polyamide 6 oligomer aqueous solution (C) or using the polyamide 6 oligomer aqueous solution (C) and the water (B) together can be preferably exemplified by a pressure higher than the saturated vapor pressure. As the water, water in a liquid state or water in a gas state such as water vapor or both may be used, but the pressure of water is preferably higher than the saturated vapor pressure because the reaction is more likely to proceed in a liquid state than in a gas state in a reaction field. Further, the upper limit of the pressure of water is not particularly limited, but can be exemplified by 20 MPa or less. Such a pressure range is preferable because the ion product of the water tends to increase. In order to bring the water into such a pressure range, a method of pressurizing the inside of a pressure vessel and sealing the pressure vessel can be mentioned. In order to pressurize the inside of the pressure vessel, a gas may be enclosed in addition to the polyamide 6 oligomer aqueous solution (C), or the polyamide 6 oligomer aqueous solution (C) and the water (B), and examples of such a gas include air, argon, and nitrogen, but it is preferable to use nitrogen or argon from the viewpoint of preventing side reactions such as an oxidation reaction. The degree of gas pressurization is not particularly limited because it is set to achieve a target pressure, but may be 0.3 MPa or more.

The amount of water used in the method for producing ε-caprolactam of the present invention is not particularly limited, but it can be preferably exemplified that the amount of water used is adjusted so that the product of X and Y satisfies the condition of 2,000 or less when the mass ratio of water to the sum of polyamide 6 and the polyamide 6 oligomer is represented by X : 1, and the reaction temperature is represented by Y°C. It can be exemplified that the product of X and Y more preferably satisfies the condition of 1,600 or less, still more preferably satisfies the condition of 1,300 or less, and particularly preferably satisfies the condition of 1,200 or less. Further, the lower limit of the product of X and Y is not particularly limited, but can be exemplified by the condition of preferably 300 or more, more preferably 320 or more, and particularly preferably the condition of 340 or more. The present invention relates to production of ε-caprolactam in an energy saving manner from a polyamide 6 resin composition aiming at achieving both circulation utilization of fossil resources and reduction in global warming gas emissions. Since water has a specific heat capacity of 4.3 kJ/kg·K and a vaporization heat of 2,250 kJ/kg, which are very high as compared with other organic solvents, it is important to reduce the amount of water used, and when the product of X and Y is in such a range, it is possible to achieve both production efficiency of ε-caprolactam and energy saving. Further, when the retention time at the reaction temperature Y°C is represented by Z minutes, the product of X, Y, and Z can be preferably exemplified by the condition of 60,000 or less. The product of X, Y, and Z more preferably satisfies the condition of 40,000 or less, still more preferably 30,000 or less, and particularly preferably 20,000 or less. Further, the lower limit of the product of X, Y, and Z is not particularly limited, but can be exemplified by preferably the condition of 5,000 or more, more preferably the condition of 8,000 or more, and particularly preferably the condition of 9,000 or more. The product of X, Y, and Z in such a preferable condition range is preferable because the production efficiency of ε-caprolactam in an energy saving manner tends to increase. In the reaction of polyamide 6 with water, in addition to the production of ε-caprolactam, a side reaction of linear oligomer production by the reaction of ε-caprolactam with water proceeds as a side reaction, and when the amount of water used is simply reduced, a large amount of linear oligomers are produced, and therefore the production efficiency of ε-caprolactam is greatly reduced. As a result of elucidating the thermodynamic equilibrium point of the production reaction of ε-caprolactam by the reaction of polyamide 6 with water in the polyamide 6 oligomer aqueous solution and the side reaction of linear oligomer production, the present inventors have found that by setting the product of X and Y and the product of X, Y, and Z within the above condition ranges, by-production of linear oligomers is prevented, and the production efficiency of ε-caprolactam is greatly improved, leading to the present invention.

In the production of ε-caprolactam of the present invention, various known reaction methods such as a batch method and a continuous method can be adopted. Examples of the batch method include an autoclave and a vertical/horizontal reactor, both having a stirrer and a heating function, and a vertical/horizontal reactor having a compression mechanism such as a cylinder in addition to a stirrer and a heating function. Examples of the continuous method include an extruder and a tubular reactor, both having a heating function, a tubular reactor having a mixing mechanism such as a baffle, a line mixer, a vertical/horizontal reactor, a vertical/horizontal reactor having a stirrer, and a tower. The atmosphere in the production is desirably a non-oxidizing atmosphere, preferably an inert atmosphere of nitrogen, helium, argon, or the like, and is preferably a nitrogen atmosphere from the viewpoint of economy and ease of handling.

### (5) Method for collecting ε-caprolactam

The method for collecting ε-caprolactam of the present invention is not particularly limited, and any method can be adopted. For example, when the depolymerization reaction is performed by a batch method, an ε-caprolactam aqueous solution is obtained by distillation together with water after completion of the depolymerization reaction. When the depolymerization reaction is performed by a continuous method, an ε-caprolactam aqueous solution can be obtained as the reaction proceeds. ε-Caprolactam with high purity can be collected by distillation of the obtained ε-caprolactam aqueous solution to separate water. In addition, if there is a component insoluble in water in the collected ε-caprolactam aqueous solution, the component is separated in advance by a known method such as solid-liquid separation and the ε-caprolactam aqueous solution can be subjected to distillation separation.

Further, as a method for obtaining ε-caprolactam with high purity, it can be combined with a purification method such as a method for precisely distilling collected ε-caprolactam, a vacuum distillation method by adding a trace amount of sodium hydroxide, an activated carbon treatment method, an ion exchange treatment method, or a recrystallization method. By these methods, impurities that are difficult to separate by distillation separation can also be efficiently removed.

### (6) Polyamide 6 and molded product thereof

In the method for producing ε-caprolactam described in the present invention, ε-caprolactam with high purity can be obtained, and therefore can be used as a polymerization raw material of polyamide 6. Polyamide 6 can be produced by a generally known method in which ε-caprolactam is subjected to hot melt polymerization in the presence of a small amount of water.

Further, the polyamide 6 thus obtained is melt-kneaded with the fibrous filling material or various additives as necessary to produce a polyamide 6 resin composition, and various molded products such as a sheet and a film can be obtained by a generally known method such as injection molding or extrusion molding.

The polyamide 6 of the present invention and a molded product thereof can be used for various applications such as electrical/electronic parts, building members, various vessels, daily necessities, household goods, and sanitary goods by taking advantage of its excellent properties. In particular, it is particularly preferably used for aircraft parts and electrical/electronic parts which require toughness and stiffness. Specifically, aircraft-related parts such as a landing gear pod, a winglet, a spoiler, an edge, a ladder, an elevator, a fairing, and a rib, and as electrical/electronic parts, for example, electrical parts such as an electrical generator, an electric motor, a transformer, a current transformer, a voltage regulator, a rectifier, a resistor, an inverter, a relay, a power contact, an electrical switch, an interrupter, a switch, a knife switch, a multi-pole rod, a motor case, a TV housing, a laptop housing and internal parts, a CRT display housing and internal parts, a printer housing and internal parts, mobile terminal housings and internal parts such as a mobile phone, a mobile personal computer, and a handheld mobile terminal, IC- and LED-compatible housings, a capacitor plate, a fuse holder, various gears, various cases, and a cabinet, and electronic parts such as a connector, an SMT-compatible connector, a card connector, a jack, a coil, a coil bobbin, a sensor, an LED lamp, a socket, a resistor, a relay, a relay case, a reflector, a small switch, a power supply part, a coil bobbin, a capacitor, a variable capacitor case, an optical pickup chassis, an oscillator, various terminal boards, a transformer, a plug, a printed circuit board, a tuner, a speaker, a microphone, a headphone, a small motor, a magnetic head base, a power module, a Si power module and a SiC power module, a semiconductor, a liquid crystal, a FDD carriage, a FDD chassis, a motor brush holder, a transformer member, a parabolic antenna, and a computer-related part, and the like are mentioned.

### [Examples]

Hereinafter, the present invention will be described with reference to examples, but the present invention is not limited by these examples.

In each example, the following raw materials were used.

### [Polyamide 6 (PA6-A)]

Polyamide 6 resin ("Amilan" (registered trademark) CM1017 manufactured by Toray Industries, Inc.), ηr = 2.70, melting point: 225°C, amount of cyclic 2- to 4-mer oligomer: 0.2 mass%

Here, the solution viscosity ηr was measured at 25°C using a 0.01 g/mL solution of 98% concentrated sulfuric acid. Further, the melting point was defined as the temperature of an endothermic peak appearing when the polyamide was cooled from a molten state to 30°C at a cooling rate of 20°C/min and then heated to the melting point + 40°C at a heating rate of 20°C/min in a nitrogen gas atmosphere using a differential scanning calorimeter. However, when two or more endothermic peaks were detected, the temperature of the endothermic peak having a highest peak intensity was defined as the melting point.

Here, the amount of the cyclic 2- to 4-mer oligomer was determined by pulverizing polyamide 6, collecting polyamide 6 powder that passed through a JIS standard sieve of 24 mesh, but did not pass through a 124 mesh, subjecting 20 g of the polyamide 6 powder to extraction with 200 mL of methanol for 3 hours using a Soxhlet extractor, and quantitatively analyzing the cyclic oligomer contained in the extract liquid using high-performance liquid chromatography. The measurement conditions are as follows.

### <Measurement conditions>

High-performance liquid chromatograph: Waters' 600E
Column: GL Sciences' ODS-3
Detector: Waters' 484 Tunable Absorbance Detector
Detection wavelength: 254 nm
Solvent: methanol/water (the composition of methanol water is 20 : 80 → 80 : 20 gradient analysis)
Flow rate: 1 mL/min

### [Fibrous filling material]

Glass fibers (T-249 manufactured by Nippon Electric Glass Co., Ltd.)

### [Alkali metal halide]

Potassium iodide (manufactured by Tokyo Chemical Industry Co., Ltd.)

### [Reference Example 1] Potassium iodide-containing glass fiber-reinforced polyamide 6 (PA6-B)

Potassium iodide-containing glass fiber-reinforced polyamide 6 (PA6-B) was prepared by blending polyamide 6, potassium iodide, and glass fibers so that the mass ratio of the polyamide 6, potassium iodide, and the glass fibers was 70/0.2/30, and supplying the polyamide 6 and potassium iodide from a main feeder, supplying the glass fibers from a side feeder, and pelletizing the extruded strings using a twin-screw extruder (TEX30α manufactured by The Japan Steel Works, Ltd.) set at a cylinder set temperature of 250°C and a screw rotation speed of 150 rpm.

### [Reference Example 2] Waste I of polyamide 6 resin molded body (PA6-C)

A polyamide 6 resin containing 30 mass% of glass fibers ("Amilan" (registered trademark) CM1011G-30 manufactured by Toray Industries, Inc.) was molded into a dumbbell piece, and then crushed to obtain a polyamide 6 molded body waste I (PA6-C) in a pellet form.

### [Reference Example 3] Waste II of polyamide 6 resin molded body (PA6-D)

A fastener part made of non-reinforced polyamide 6 (polyamide 6 content: 99 mass% or more) was collected and crushed to obtain a polyamide 6 molded body waste II (PA6-D). As a result of the high-performance liquid chromatography analysis described above, the amount of a cyclic 2- to 4-mer oligomer in polyamide 6 in the polyamide molded body waste II was 0.4 mass%.

### [Reference Example 4] Production of polyamide 6 oligomer

In an autoclave made of SUS316L equipped with a stirrer, 20.0 g of the polyamide 6 (PA6-A) and 60.0 g of deionized water were charged. The mass ratio (X : 1) of water to polyamide 6 is 3 : 1.

The reaction vessel was purged with nitrogen and sealed under a nitrogen pressure of 0.5 MPa, and then the reaction was carried out by holding the reaction vessel at 320°C for 15 minutes with stirring at 200 rpm. After completion of the reaction, the reaction mixture was cooled to room temperature and collected. Since the reaction temperature Y°C is 320°C, the product of X and Y is 960, and since the retention time at the reaction temperature of 320°C is 15 minutes, the product of X, Y, and Z is 14,400.

The yield of ε-caprolactam calculated by high-performance liquid chromatography measurement of the collected reaction mixture was 78%.

To the obtained reaction mixture, 10 times the amount (mass) of methanol was added, and the mixture was stirred to form a slurry, and then filtered through a glass filter (average pore diameter: 10 to 16 µm) to obtain a solid component. Further, an operation in which 5 times the amount of methanol is added to the obtained solid component, and the mixture was stirred to form a slurry, and then filtered through a glass filter was repeated 3 times, and then the filter residue was subjected to vacuum drying at 50°C for 12 hours to obtain a polyamide 6 oligomer. The obtained polyamide 6 oligomer was subjected to high-performance liquid chromatography analysis and found that the amount of a linear 2- to 12-mer oligomer is 95.8 mass%. High performance liquid chromatography measurement conditions here are as follows.
Apparatus: LC-10Avp series manufactured by Shimadzu Corporation
Column: Mightysil RP-18GP 150-4.6
Detector: Photodiode array detector (UV = 205 nm)
Flow rate: 1 mL/min
Column temperature: 40°C
Mobile phase: 0.1% formic acid aqueous solution/0.1% formic acid acetonitrile solution
Polyamide 6 oligomer composition: the amount of a linear 2- to 12-mer oligomer in the polyamide 6 oligomer is calculated from the peak area ratio of each polyamide 6 oligomer

### <<Evaluation Method>>

### [Yield of ε-caprolactam (HPLC)]

The calculation of the yield of ε-caprolactam (HPLC) of the present invention was performed by high-performance liquid chromatography measurement. The measurement conditions are as follows.
Apparatus: LC-10Avp series manufactured by Shimadzu Corporation
Column: Mightysil RP-18GP 150-4.6
Detector: Photodiode array detector (UV = 205 nm)
Flow rate: 1 mL/min
Column temperature: 40°C
Mobile phase: 0.1% acetic acid aqueous solution/acetonitrile
Sample: A high-performance liquid chromatography analysis sample was prepared by taking about 0.1 g of the reaction mixture, diluting it with about 10 g of deionized water, and separating and removing components insoluble in deionized water by filtration.
Quantification of ε-caprolactam: The amount of ε-caprolactam with respect to polyamide 6 was quantified by an absolute calibration curve method.

### [Example 1]

In an autoclave made of SUS316L equipped with a stirrer, 17.6 g of the polyamide 6 (PA6-A) and 25.5 g of deionized water were weighed, and 36.9 g of a polyamide 6 oligomer aqueous solution having a concentration of 6.5 mass% was further added thereto. The mass ratio (X : 1) of water to the polyamide 6 and the polyamide 6 oligomer is 3 : 1. The polyamide 6 oligomer used here is the polyamide 6 oligomer described in Reference Example 4.

The reaction vessel was purged with nitrogen and sealed under a nitrogen pressure of 0.5 MPa, and then the reaction was carried out by holding the reaction vessel at 320°C for 15 minutes with stirring at 200 rpm. The ultimate pressure during the reaction was 10.5 MPa. After completion of the reaction, the reaction mixture was cooled to room temperature and collected. Since the reaction temperature Y°C is 320°C, the product of X and Y is 960, and since the retention time at the reaction temperature of 320°C is 15 minutes, the product of X, Y, and Z is 14,400.

The production amount of ε-caprolactam calculated by high-performance liquid chromatography measurement of the collected reaction mixture was 15.8 g, and the yield with respect to the polyamide 6 (PA6-A) used in the reaction was 89.8%.

Further, the reaction mixture collected by the reaction was subjected to distillation separation of water at a reduced pressure of 30 mmHg and a heating temperature of 55°C to obtain a concentrated ε-caprolactam aqueous solution, and further distilled at a reduced pressure of 5 mmHg and a heating temperature of 150 to 170°C to obtain distilled ε-caprolactam. The concentration and distillation yield was 95.8%. The HPLC impurity in the distilled ε-caprolactam was 0.48%, and the quality was such that it can be used as a polymerization raw material of polyamide 6.

Comparison with Reference Example 4 in which the reaction was carried out by bringing polyamide 6 and water into contact with each other shows that the yield of ε-caprolactam with respect to polyamide 6 used as a raw material is significantly improved by further adding a polyamide 6 oligomer aqueous solution and performing the reaction when polyamide 6 and water are brought into contact with water.

### [Reference Example 5]

A reaction mixture was prepared by performing a reaction under the same conditions as in Example 1. To the obtained reaction mixture, about 10 times the amount (mass) of methanol was added, and the mixture was stirred to form a slurry, and then filtered through a glass filter (average pore diameter: 10 to 16 µm) to collect a solid component. Further, an operation in which about 5 times the amount of methanol is added to the collected solid component, and the mixture was stirred to form a slurry, and then filtered through a glass filter was repeated 4 times, and then the filter residue was subjected to vacuum drying at 50°C for 12 hours to collect a polyamide 6 oligomer. The collected polyamide 6 oligomer was subjected to high-performance liquid chromatography measurement under the conditions described in [Reference Example 4] and found to be a polyamide 6 oligomer with high purity containing 96.8 mass% of a linear 2- to 12-mer oligomer.

### [Example 2]

In an autoclave made of SUS316L equipped with a stirrer, 17.6 g of the polyamide 6 (PA6-A) and 25.5 g of deionized water were weighed, and 36.9 g of a polyamide 6 oligomer aqueous solution prepared to a concentration of 6.5 mass% using a polyamide 6 oligomer prepared by the method described in Reference Example 5 was further added thereto. The mass ratio (X : 1 of water to the polyamide 6 and the polyamide 6 oligomer is 3 : 1.

The reaction vessel was purged with nitrogen and sealed under a nitrogen pressure of 0.5 MPa, and then the reaction was carried out by holding the reaction vessel at 320°C for 15 minutes with stirring at 200 rpm. After completion of the reaction, the reaction mixture was cooled to room temperature and collected. Since the reaction temperature Y°C is 320°C, the product of X and Y is 960, and since the retention time at the reaction temperature of 320°C is 15 minutes, the product of X, Y, and Z is 14,400.

The production amount of ε-caprolactam calculated by high-performance liquid chromatography measurement of the collected reaction mixture was 15.7 g, and the yield with respect to the polyamide 6 (PA6-A) used in the reaction was 89.2%.

Comparison between Example 1 and Example 2 shows that the use of a polyamide 6 oligomer prepared by a different method does not affect the yield of ε-caprolactam with respect to polyamide 6 used in the reaction.

### [Example 3]

In an autoclave made of SUS316L equipped with a stirrer, 25.1 g of PA6-B prepared in Reference Example 1 and 25.5 g of deionized water were weighed, and 36.9 g of a polyamide 6 oligomer aqueous solution having a concentration of 6.5 mass% was further added thereto. The mass ratio (X : 1) of water to the polyamide 6 and the polyamide 6 oligomer is 3 : 1. The polyamide 6 oligomer used here is the polyamide 6 oligomer described in Reference Example 4.

The reaction vessel was purged with nitrogen and sealed under a nitrogen pressure of 0.5 MPa, and then the reaction was carried out by holding the reaction vessel at 340°C for 15 minutes with stirring at 200 rpm. After completion of the reaction, the reaction mixture was cooled to room temperature and collected. Since the reaction temperature Y°C is 340°C, the product of X and Y is 1,020, and since the retention time at the reaction temperature of 340°C is 15 minutes, the product of X, Y, and Z is 15,300.

The production amount of ε-caprolactam calculated from high-performance liquid chromatography measurement of the collected reaction mixture was 15.0 g, and the yield with respect to polyamide 6 in PA6-B in PA6-B used in the reaction was 85.4%.

### [Example 4]

In an autoclave made of SUS316L equipped with a stirrer, 25.1 g of PA6-C described in Reference Example 2 and 25.5 g of deionized water were weighed, and 36.9 g of a polyamide 6 oligomer aqueous solution having a concentration of 6.5 mass% was further added thereto. The mass ratio (X : 1) of water to the polyamide 6 and the polyamide 6 oligomer is 3 : 1. The polyamide 6 oligomer used here is the polyamide 6 oligomer described in Reference Example 4.

The reaction vessel was purged with nitrogen and sealed under a nitrogen pressure of 0.5 MPa, and then the reaction was carried out by holding the reaction vessel at 320°C for 15 minutes with stirring at 200 rpm. After completion of the reaction, the reaction mixture was cooled to room temperature and collected. Since the reaction temperature Y°C is 320°C, the product of X and Y is 960, and since the retention time at the reaction temperature of 320°C is 15 minutes, the product of X, Y, and Z is 14,400.

The production amount of ε-caprolactam calculated by high-performance liquid chromatography measurement of the collected reaction mixture was 14.9 g, and the yield with respect to the polyamide 6 in PA6-C used in the reaction was 84.8%.

### [Example 5]

A reaction mixture was prepared by performing a reaction under the same conditions as in Example 1 except that PA6-D described in Reference Example 3 was used in place of PA6-A.

The production amount of ε-caprolactam calculated by high-performance liquid chromatography of the collected reaction mixture was 15.7 g, and the yield with respect to the polyamide 6 in PA6-D used in the reaction was 89.2%.

### [Example 6] Polymerization of polyamide 6

In a test tube, 10 g of ε-caprolactam collected by the method described in Example 1, 2.2 mg of benzoic acid, and 10.0 g of ion exchanged water were weighed. The test tube was placed in an autoclave, the inside of the autoclave was purged with nitrogen, and then the jacket temperature was set to 250°C, and heating was started. After the internal pressure reached 1.0 MPa, the internal pressure was maintained at 1.0 MPa for 3 hours. Thereafter, the internal pressure was released to normal pressure over 1.5 hours, and heating was stopped when the internal temperature reached 228°C. After completion of the polymerization, the polymer was collected from the test tube and subjected to a crushing treatment. The crushed polymer was treated in hot water at 95°C for 15 hours to extract and remove unreacted monomers and low polymers. The polymer after extraction was subjected to vacuum drying at 80°C for 24 hours to obtain a polyamide 6 resin having a melting point of 225°C and ηr = 2.69.

### [Example 7]

In an autoclave made of SUS316L equipped with a stirrer, 39.6 g of the polyamide 6 (PA6-A) and 57.4 g of deionized water were weighed, and 83.0 g of a polyamide 6 oligomer aqueous solution prepared to a concentration of 6.5 mass% using a polyamide 6 oligomer prepared by the method described in Reference Example 4 was further added thereto. The mass ratio (X : 1 of water to the polyamide 6 and the polyamide 6 oligomer is 3 : 1).

The reaction vessel was purged with nitrogen and sealed under a nitrogen pressure of 5.0 MPa, and then the reaction was carried out by holding the reaction vessel at 320°C for 15 minutes with stirring at 200 rpm. The ultimate pressure during the reaction was 19.7 MPa.

After completion of the reaction, the reaction mixture was cooled to room temperature and collected. Since the reaction temperature Y°C is 320°C, the product of X and Y is 960, and since the retention time at the reaction temperature of 320°C is 15 minutes, the product of X, Y, and Z is 14,400.

The production amount of ε-caprolactam calculated by high-performance liquid chromatography measurement of the collected reaction mixture was 36.9 g, and the yield with respect to the polyamide 6 (PA6-A) used in the reaction was 93.2%.

Comparison with Example 1 shows that there is a tendency that ε-caprolactam is obtained in high yield by setting the pressure during the reaction to a high pressure equal to or higher than the saturated vapor pressure.

### [Example 8]

In an autoclave made of SUS316L equipped with a stirrer, 26.4 g of the polyamide 6 (PA6-A) and 25.5 g of deionized water were weighed, and 36.9 g of a polyamide 6 oligomer aqueous solution prepared to a concentration of 9.8 mass% using a polyamide 6 oligomer prepared by the method described in Reference Example 4 was further added thereto. The mass ratio (X : 1 of water to the polyamide 6 and the polyamide 6 oligomer is 2 : 1).

The reaction vessel was purged with nitrogen and sealed under a nitrogen pressure of 0.5 MPa, and then the reaction was carried out by holding the reaction vessel at 320°C for 15 minutes with stirring at 200 rpm.

After completion of the reaction, the reaction mixture was cooled to room temperature and collected. Since the reaction time Y°C is 320°C, the product of X and Y is 640, and since the retention time at the reaction temperature of 320°C is 15 minutes, the product of X, Y, and Z is 9,600.

The production amount of ε-caprolactam calculated by high-performance liquid chromatography measurement of the collected reaction mixture was 18.8 g, and the yield with respect to the polyamide 6 (PA6-A) used in the reaction was 71.0%.

### [Example 9]

In an autoclave made of SUS316L equipped with a stirrer, 52.6 g of the polyamide 6 (PA6-A) and 51.0 g of deionized water were weighed, and 73.8 g of a polyamide 6 oligomer aqueous solution prepared to a concentration of 9.8 mass% using a polyamide 6 oligomer prepared by the method described in Reference Example 4 was further added thereto. The mass ratio (X : 1 of water to the polyamide 6 and the polyamide 6 oligomer is 2 : 1).

The reaction vessel was purged with nitrogen and sealed under a nitrogen pressure of 5.0 MPa, and then the reaction was carried out by holding the reaction vessel at 320°C for 15 minutes with stirring at 200 rpm. The ultimate pressure during the reaction was 19.8 MPa.

After completion of the reaction, the reaction mixture was cooled to room temperature and collected. Since the reaction time Y°C is 320°C, the product of X and Y is 640, and since the retention time at the reaction temperature of 320°C is 15 minutes, the product of X, Y, and Z is 9,600.

The production amount of ε-caprolactam calculated by high-performance liquid chromatography measurement of the collected reaction mixture was 45.1 g, and the yield with respect to the polyamide 6 (PA6-A) used in the reaction was 85.7%.

Comparison with Example 8 shows that there is a tendency that ε-caprolactam is obtained in high yield by setting the pressure during the reaction to a high pressure equal to or higher than the saturated vapor pressure.

### [Reference Example 6] Hot water extraction of polyamide 6

In a 70-liter autoclave, 20 kg of ε-caprolactam, 4.32 g of benzoic acid, and 3.0 kg of ion exchanged water were charged, the inside of the polymerization can was sealed and sufficiently purged with nitrogen, and then the temperature was raised until the pressure inside the can of the polymerization reactor reached 0.98 MPa with stirring, and the temperature was continuously raised to 250°C while the pressure inside the can was maintained. After the temperature reached 250°C, the pressure was released to atmospheric pressure over 40 minutes. Thereafter, the mixture was held at 250°C for 180 minutes at atmospheric pressure, and then the polyamide 6 polymer was discharged and cooled/cut to form a pellet.

This pellet was subjected to extraction using 20 times the amount of hot water at 98°C to collect an extract liquid containing unreacted caprolactam and a polyamide 6 oligomer. The total amount of the unreacted caprolactam and the polyamide 6 oligomer in the extract liquid was 0.5 mass%, and the amount of the polyamide 6 oligomer was 0.1 mass%.

### [Example 10]

The hot water extract liquid in the PA6 production step obtained by the method described in Reference Example 6 was concentrated until the concentration of unreacted caprolactam and the polyamide 6 oligomer reached 6.5 mass%, and depolymerization was performed using the obtained concentrated liquid.

In an autoclave made of SUS316L equipped with a stirrer, 17.6 g of the polyamide 6 (PA6-A) and 25.5 g of deionized water were weighed, and 36.9 g of the concentrated liquid was further added thereto. The mass ratio (X : 1) of water to the polyamide 6 and the polyamide 6 oligomer is 3.3 : 1.

The reaction vessel was purged with nitrogen and sealed under a nitrogen pressure of 0.5 MPa, and then the reaction was carried out by holding the reaction vessel at 320°C for 15 minutes with stirring at 200 rpm. After completion of the reaction, the reaction mixture was cooled to room temperature and collected. Since the reaction temperature Y°C is 320°C, the product of X and Y is 1056, and since the retention time at the reaction temperature of 320°C is 15 minutes, the product of X, Y, and Z is 15,840.

The production amount of ε-caprolactam calculated by high-performance liquid chromatography measurement of the collected reaction mixture was 14.8 g, and the yield with respect to the polyamide 6 (PA6-A) used in the reaction was 84.1%.

### [Example 11]

The hot water extract liquid in the PA6 production step obtained by the method described in Reference Example 6 was concentrated until the total concentration of unreacted caprolactam and the polyamide 6 oligomer reached 6.5 mass%, and depolymerization was performed using the obtained concentrated liquid.

In an autoclave made of SUS316L equipped with a stirrer, 20.0 g of the polyamide 6 (PA6-A) and 65.0 g of the concentrated liquid were added. The mass ratio (X : 1) of water to the polyamide 6 and the polyamide 6 oligomer is 2.9 : 1.

The reaction vessel was purged with nitrogen and sealed under a nitrogen pressure of 0.5 MPa, and then the reaction was carried out by holding the reaction vessel at 320°C for 15 minutes with stirring at 200 rpm. After completion of the reaction, the reaction mixture was cooled to room temperature and collected. Since the reaction temperature Y°C is 320°C, the product of X and Y is 928, and since the retention time at the reaction temperature of 320°C is 15 minutes, the product of X, Y, and Z is 13,920.

The production amount of ε-caprolactam calculated by high-performance liquid chromatography measurement of the collected reaction mixture was 15.7 g, and the yield with respect to the polyamide 6 (PA6-A) used in the reaction was 78.5%.

## Claims

1. A method for producing ε-caprolactam, comprising bringing a resin composition (A) containing at least polyamide 6 and a polyamide 6 oligomer aqueous solution (C) heated to 290°C or higher and 350°C or lower into contact with each other, or bringing a resin composition (A) containing at least polyamide 6, a polyamide 6 oligomer aqueous solution (C) heated to 290°C or higher and 350°C or lower, and further water (B) heated to 290°C or higher and 350°C or lower into contact with each other.

2. The method for producing ε-caprolactam according to claim 1, wherein the polyamide 6 oligomer aqueous solution (C) is further added in a method for producing ε-caprolactam by adding the resin composition (A) containing at least polyamide 6 and the water (B) heated to 290°C or higher and 350°C or lower to make contact with each other.

3. The method for producing ε-caprolactam according to claim 1 or 2, wherein a polyamide 6 oligomer used in the polyamide 6 oligomer aqueous solution (C) is a polyamide 6 oligomer by-produced in the method for producing ε-caprolactam.

4. The method for producing ε-caprolactam according to any one of claims 1 to 3, wherein an amount of a linear 2-to 12-mer oligomer contained in the polyamide 6 oligomer is 90 mass% or more.

5. The method for producing ε-caprolactam according to any one of claims 1 to 4, wherein the contact is made under a condition that a product of X and Y is 2,000 or less when a mass ratio of water to a sum of polyamide 6 and a polyamide 6 oligomer used in the method for producing ε-caprolactam is represented by X : 1 and a reaction temperature is represented by Y°C.

6. The method for producing ε-caprolactam according to any one of claims 1 to 5, wherein the resin composition (A) containing at least polyamide 6 contains an alkali metal halide.

7. The method for producing ε-caprolactam according to any one of claims 1 to 6, wherein the polyamide 6 oligomer aqueous solution (C) is an extract liquid obtained in a step of extracting a polyamide 6 oligomer with hot water from polyamide 6 which is a product at a time of production of polyamide 6.

8. The method for producing ε-caprolactam according to any one of claims 1 to 7, wherein the resin composition (A) containing at least polyamide 6 is a waste of a resin molded body containing at least polyamide 6.

9. A method for producing polyamide 6, comprising obtaining ε-caprolactam by the method according to any one of claims 1 to 8, and polymerizing polyamide 6.
